# EUROPÄISCHE PATENTSCHRIFT

(11) **EP 3 090 710 B1**
(45) Veröffentlichungstag und Bekanntmachung des Hinweises auf die Patenterteilung: **12.07.2017**
(21) Anmeldenummer: 15166806.8
(22) Anmeldetag: 07.05.2015
(51) Int. Cl.: A61F 13/49, B32B 5/04, B32B 7/14, B32B 25/08, B32B 27/08, B32B 37/12, B32B 25/10, B32B 27/12

(54) **ELASTISCHE WINDELKOMPONENTE**
ELASTIC DIAPER COMPONENT
COMPOSANT DE COUCHE ÉLASTIQUE

(43) Veröffentlichungstag der Anmeldung: 09.11.2016
(73) Patentinhaber: Mondi AG, 1030 Wien (AT)
(72) Erfinder: Schönbeck, Marcus, 33775 Versmold (DE)
(74) Vertreter: Lorenz, Bernd Ingo Thaddeus

(56) Entgegenhaltungen:
- EP-A1- 2 106 776
- EP-A1- 2 158 888
- WO-A1-2006/124337
- WO-A1-2008/021701

## Beschreibung

Die Erfindung betrifft eine elastische Windelkomponente bestehend aus einem Laminat, welches zumindest eine Trägerschicht und eine mit der Trägerschicht verklebte elastische Folie aufweist und zumindest im Bereich der elastischen Folie in Zugrichtung elastisch dehnbar ist. Das Laminat weist ein erstes Anschlussende zur Befestigung an einer Windel und ein zweites Anschlussende für ein Verschlusselement auf, wobei das zweite Anschlusselement eine geringere Breite quer zur Zugrichtung hat als das erste Anschlussende. In einem elastischen Bereich zwischen den beiden Anschlussenden ist die Trägerschicht mit der elastischen Folie durch eine Mehrzahl paralleler Klebstoffstreifen, die sich quer zur Zugrichtung erstrecken, verbunden. Eine solche Windelkomponente wird in der Praxis häufig auch als Windelohr (back ear laminat) bezeichnet.

Eine elastische Windelkomponente mit den vorgenannten Merkmalen ist beispielsweise aus WO 2006/124337 A1 bekannt. Der Klebstoff zur Verbindung der elastischen Folie mit der zum Beispiel aus einem Nonwoven bestehenden Trägerschicht bildet ein Klebstoffmuster aus einer Vielzahl paralleler Klebstoffstreifen, die sich quer zur Zugrichtung erstrecken. Im Vergleich zu einer flächigen Verklebung bewirkt das beschriebene Klebstoffmuster eine Verbesserung der elastischen Dehnungseigenschaften des Laminats. Üblicherweise wird der elastische Bereich der Windelkomponente durch eine Verstreckung mechanisch aktiviert. Die streifenförmige Applikation des Klebstoffes verbessert die Aktivierbarkeit und die Wirksamkeit der Aktivierung. Im Rahmen der bekannten Maßnahmen weisen die Klebstoffstreifen des Klebstoffmusters dieselbe Breite auf und sind in einem vorgegebenen konstanten Abstand angeordnet. Gemäß WO 2006/124337 A1 stehen die Breite der Klebstoffstreifen und der Abstand zwischen den Streifen in einem festen Verhältnis, das in einem Bereich zwischen 0,33 und 1,0 festgelegt wird.

Die EP 2 158 888 A1 betrifft eine Laminatbahn, aus der in einem mehrnutzigen Stanzverfahren Windel komponenten ausgestanzt werden können. Die Laminatbahn weist eine elastische Trägerfolie und Deckschichten aus Nonwoven auf, wobei die Lagen miteinander verklebt sind. Die Laminatbahn weist flächig miteinander verklebte Bereiche und streifenförmig verklebte Bereiche auf. Die Klebstoffstreifen bilden ein Muster, welches unterschiedlich breite und/oder mit unterschiedlichen Abständen angeordnete Klebstoffstreifen umfasst. Das Klebstoffmuster nimmt dabei keine Rücksicht auf die Form der aus der Laminatbahn ausgestanzten Windelkomponenten.

Die vorbekannten Laminate zeichnen sich durch zufriedenstellende Dehnungseigenschaften aus, sofern die Dehnungsmessungen an einem Laminatstreifen konstanter Breite durchgeführt werden. Bei einem Zugversuch an Prüfstücken, die hinsichtlich ihrer Abmessungen üblicherweise normiert sind, können einheitliche Dehnungseigenschaften gemessen werden.

Bei Windelkomponenten, die als sogenannte Windelohren konfektioniert sind und ein erstes breites Anschlussende zur Befestigung an einer Windel und ein schmales zweites Anschlussende für ein Verschlusselement aufweisen, wurde beobachtet, dass die Dehnung bei einer Verstreckung des Laminats in Zugrichtung nicht gleichmäßig ist. An dem schmalen Anschlussende ist der elastische Bereich der Windelkomponente einer größeren Zugbeanspruchung ausgesetzt und dehnt sich stärker als der an das breite Anschlussende angrenzende elastische Bereich des Laminats. Nahe des breiten Anschlussendes wird eine bessere Dehnung benötigt, während an dem zweiten Anschlussende eine höhere Festigkeit anzustreben ist.

Vor diesem Hintergrund liegt der Erfindung die Aufgabe zugrunde, eine bessere Verteilung der Performance der elastischen Windelkomponente zwischen den beiden Anschlussenden zu erreichen. Insbesondere wird angestrebt, dass die Dehnungseigenschaften einer Windelkomponente, welche ein erstes breites Anschlussende und ein zweites schmales Anschlussende aufweist, im elastischen Bereich einheitlich sind, so dass sich der elastische Bereich der Windelkomponente nahe des ersten Anschlussendes ebenso stark ausdehnt wie in der Nähe des zweiten Abschlusselementes, wenn die Windelkomponente im Gebrauch gedehnt wird.

Gegenstand der Erfindung und Lösung dieser Aufgabe ist eine elastische Windelkomponente nach Anspruch 1.

Erfindungsgemäß bilden die Klebstoffstreifen ein Klebstoffmuster, welches in einem an das zweite Anschlussende angrenzenden Bereich breitere Klebstoffstreifen und/oder einen geringeren Abstand zwischen benachbarten Klebstoffstreifen aufweist als in einem an das erste Anschlussende angrenzenden Bereich. Die Breite der Klebstoffstreifen und/oder der Abstand zwischen den Klebstoffstreifen ist variabel und richtet sich nach der jeweiligen Breite des Laminats. Die Klebstoffverteilung wird erfindungsgemäß so festgelegt, dass bei einer Verstreckung des Laminats in Zugrichtung in der Nähe des an einer Windel zu befestigenden ersten Anschlussendes eine in etwa ebenso große Dehnung erreicht wird, wie in der Nähe des zweiten, schmaleren Anschlussendes. Es resultiert eine bessere Verteilung der Performance der Windelkomponente.

Der Flächenanteil der aus den Klebstoffstreifen gebildeten Klebefläche bezogen auf die aus der Klebefläche und den klebstofffreien Flächen gebildeten Grundfläche ändert sich in Abhängigkeit der Breite des Laminats, wobei der Flächenanteil der Klebstofffläche mit kleiner werdender Laminatbreite zunimmt.

Erfindungsgemäß sind die Breite der Klebstoffstreifen und/oder der Abstand zwischen benachbarten Klebstoffstreifen in Abhängigkeit der Breite des Laminats so festgelegt, dass die Dehnung des elastischen Bereiches bei einer Verstreckung des Laminats in Zugrichtung im Wesentlichen einheitlich ist.

Die Klebstoffstreifen können insbesondere eine Breite zwischen 0,5 mm und 1,2 mm aufweisen. Der Abstand zwischen den Klebstoffstreifen kann zwischen 0,5 mm und 2,0 mm variieren. Gemäß einer bevorzugten Ausführung der Erfindung weisen die Klebstoffstreifen eine konstante Breite zwischen 0,5 mm und 1,2 mm auf, während der Abstand zwischen den Klebstoffstreifen nicht konstant ist und einen in Abhängigkeit der Breite des Laminats festgelegten Wert zwischen 0,5 mm und 2,0 mm aufweist. Alternativ kann der Abstand zwischen den Klebstoffstreifen konstant sein und die Breite der Klebstoffstreifen in Abhängigkeit der jeweiligen Laminatbreite variiert werden.

Als Klebstoff für die Klebeverbindung zwischen der elastischen Folie und der Trägerschicht werden vorzugsweise Schmelzklebstoffe (hot melt Klebstoffe) eingesetzt. Geeignet sind beispielsweise Klebstoffe auf der Basis von Styrol-Olefin-Styrol-Block-Copolymeren.

Der elastische Bereich des Laminats ist vorzugsweise mechanisch aktiviert. Die mechanische Aktivierung des Laminats folgt zweckmäßig durch einen sogenannten Ringrollprozess, bei dem die Laminatbahn eine Profilwalzenanordnung durchläuft und das Laminat im Walzenpalt zwischen den Profilwalzen lokal überstreckt wird. Die mechanische Aktivierung ist an dem fertigen Produkt sichtbar in Form sogenannter Aktivierungsstreifen. Die Aktivierungsstreifen sind alternierend angeordnete streifenförmige Zonen, in denen das Material einer Überstreckung unterworfen wurde und Zonen, in denen eine geringere oder keine Überstreckung erfolgte. Die Aktivierungsstreifen erstrecken sich quer Zugrichtung und verlaufen parallel zu den Klebstoffstreifen.

Die Trägerschicht besteht vorzugsweise aus einem Nonwoven. Hinsichtlich des Fasermaterials und der Art der Verfestigung unterliegt das Nonwovenmaterial keinen Beschränkungen. Als Fasermaterialien kommen beispielsweise Polyolefine, insbesondere Polyethylen und Polypropylen, Polyester, Polyamid, Cellulose sowie Bikomponentenfasern und Kombinationen aus den vorgenannten Fasermaterialien in Betracht. Das Nonwoven kann insbesondere ein Flächengewicht zwischen 8 g/m² und 50 g/m² aufweisen.

Die elastische Folie besteht aus einem thermoplastischen Elastomer, wobei insbesondere Polymere aus der Gruppe der Styrol-Butadien-Styrol-Block-Copolymere (SBS), Styrol-Isopren-Styrol-Block-Copolymere (SIS), Styrol-Ethylen-Butadien-Styrol-Block-Copolymere (SEBS), der elastischen Polyethylen-Copolymere, elastischen Polypropylen-Copolymere, elastischen Polyurethan-Copolymere, elastischen Polyamid-Copolymere sowie Mischungen dieser Polymere geeignet sind. Neben einer Verwendung von Monofolien können auch coextrudierte Folien eingesetzt werden, wobei coextrudierte Folien mit mehreren identischen Schichten ebenfalls geeignet sind. Die elastische Folie kann insbesondere eine Dicke zwischen 10 µm und 130 µm aufweisen.

Gemäß einer bevorzugten Ausführung der Erfindung ist die elastische Folie zwischen zwei aus Nonwoven bestehenden Trägerschichten angeordnet und mit diesen verklebt, wobei die Klebeverbindungen auf beiden Seiten der elastischen Folie in der zuvor beschriebenen Weise ausgeführt sind.

Die Anschlussenden des Laminats zur Befestigung an einer Windel und zum Anschluss eines Verschlusselements sind zweckmäßig als nichtelastische Zonen ausgebildet. Das erste Anschlussende zur Befestigung an einer Windel ist zweckmäßig weich und flexibel ausgebildet. Sofern die Windelkomponente eine elastische Folie aufweist, die zwischen zwei Nonwovenschichten einkaschiert ist, endet die elastische Folie vorzugsweise vor dem ersten Anschlussende und setzt sich das erste Anschlussende lediglich aus den beiden miteinander verbundenen Nonwovenschichten zusammen. Vorzugsweise sind die beiden Nonwovenschichten an dem ersten Anschlussende flächig miteinander verklebt. Das zweite Anschlussende zur Befestigung eines Verschlusselements ist vorzugsweise durch eine Verstärkungsschicht versteift, die in einem Überlappungsbereich mit der einkaschierten elastischen Folie verbunden ist. Die Verstärkungsschicht besteht vorzugsweise aus einem Polymer, welches im Vergleich zu der elastischen Folie eine größere Biegesteifigkeit und größere Zugfestigkeit aufweist. Im Bereich des zweiten Anschlussendes sind die einzelnen Schichten vorzugsweise flächig miteinander verklebt. Im Rahmen der Erfindung liegt es auch, dass an dem zweiten Anschlussende ein nicht elastischer Streifen mit Hakenelementen vorgesehen ist, der mit der elastischen Folie des Laminats und/oder einer beispielsweise aus Nonwoven bestehenden Trägerschicht verbunden ist.

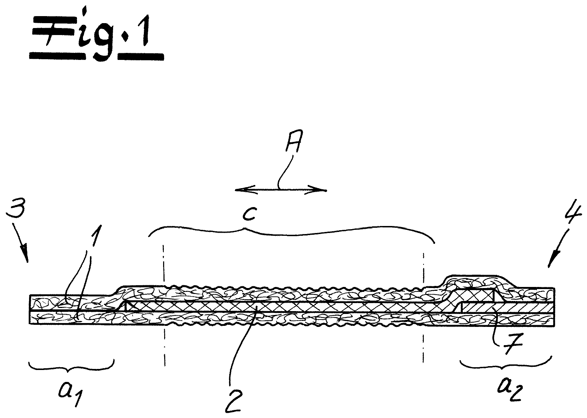

Im Folgenden wird die Erfindung anhand einer lediglich ein Ausführungsbeispiel darstellenden Zeichnung erläutert. Es zeigen schematisch
- Fig. 1: einen Längsschnitt in Dehnungsrichtung durch eine elastische Windelkomponente,
- Fig. 2: eine Draufsicht auf die in Fig. 1 dargestellte Windelkomponente,
- Fig. 3: das Klebstoffmuster einer Klebeverbindung zwischen einer Trägerschicht und einer elastischen Folie der in den Fig. 1 und 2 dargestellten Windelkomponente,
- Fig. 4: einen Ausschnitt aus Fig. 3 in einer gegenüber Fig. 3 vergrößerten Darstellung,
- Fig. 5: eine Ausführungsvariante des in Fig. 3 dargestellten Klebstoffmusters.

Die Figuren betreffen eine elastische Windelkomponente, die in der Praxis auch als Windelohr bezeichnet wird. Die Windelkomponente besteht aus einem Laminat, welches zumindest eine Trägerschicht 1 und eine mit der Trägerschicht 1 verklebte elastische Folie 2 aufweist und zumindest im Bereich der elastischen Folie 2 in Zugrichtung A elastisch dehnbar ist. In den Ausführungsbeispielen und gemäß einer bevorzugten Ausführung der Erfindung ist die elastische Folie 2 zwischen zwei Trägerschichten 1 einkaschiert und mit diesen verklebt, wobei die Trägerschichten 1 jeweils aus einem Nonwoven bestehen. Die Trägerschichten sind nicht elastisch, aber in ausreichendem Maße dehnbar.

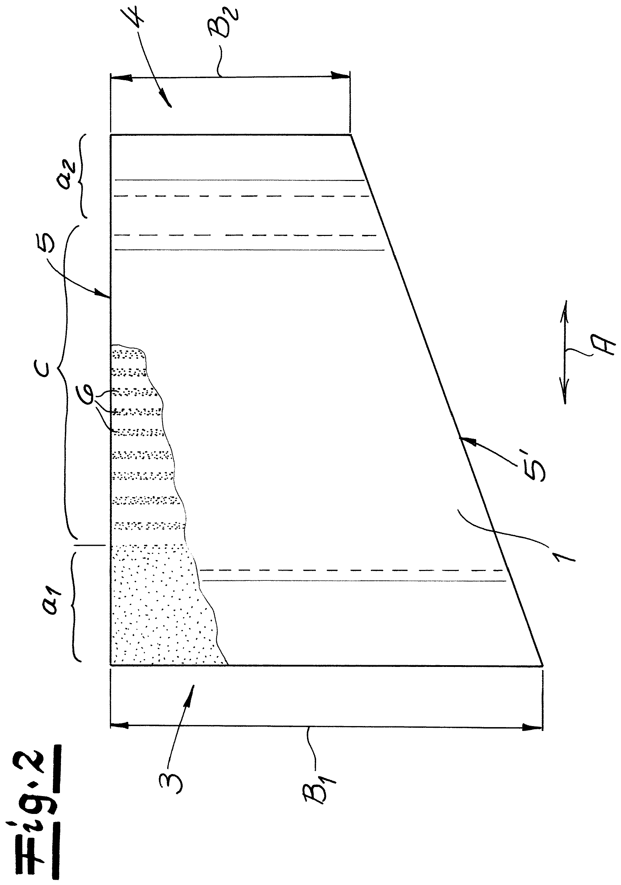

Gemäß der Darstellung in Fig. 2 weist das Laminat ein erstes Anschlussende 3 zur Befestigung an einer Windel und ein zweites Anschlussende 4 für ein Verschlusselement, z. B. ein Tape oder ein mit Haken versehenes Verschlussteil auf. Das zweite Anschlussende 4 hat eine geringere Breite B₂ quer zur Zugrichtung als das erste Anschlussende 3 mit der Breite B₁. Im Ausführungsbeispiel hat die Windelkomponente die Form eines allgemeinen Vierecks, wobei die Anschlussenden 3, 4 parallel zueinander verlaufen und die beiden Längsseiten 5, 5' zueinander schräg ausgerichtet sind. Die Windelkomponente kann auch die Grundform eines Trapezes aufweisen. Ferner können die Längsseiten 5, 5' auch bogenförmig gekrümmt sein.

Die Windelkomponente weist nicht elastische Zonen a₁, a₂ an den Anschlussenden auf. Zwischen den nicht elastischen Zonen a₁, a₂ hat die Windelkomponente einen elastischen Bereich c. In diesem elastischen Bereich c zwischen den beiden Anschlussenden 3, 4 ist die elastische Folie 2 mit den außenseitigen Trägerschichten 1 aus Nonwoven durch eine Mehrzahl paralleler Klebstoffstreifen 6 verbunden. Die Klebstoffstreifen 6 erstrecken sich quer zur Zugrichtung A.

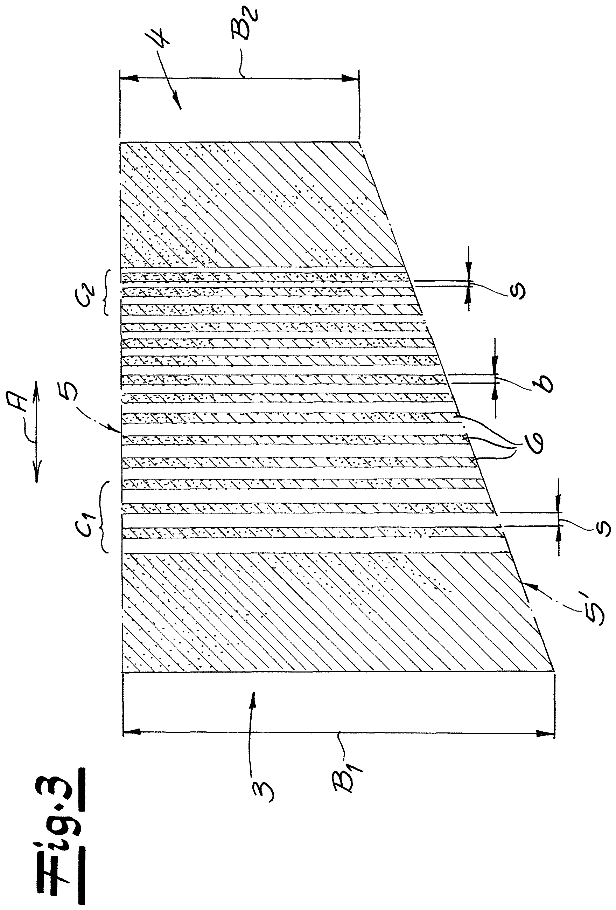

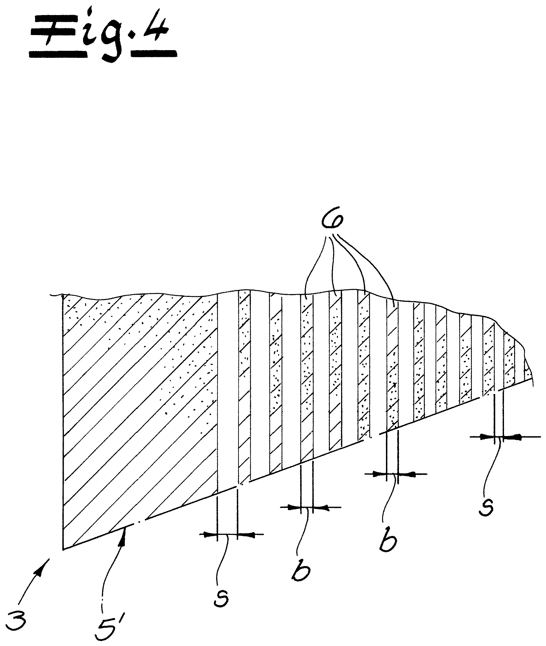

Das Klebemuster der Klebeverbindungen zwischen der elastischen Folie 2 und den beiden außenseitigen Trägerschichten 1 ist in den Fig. 3 und 4 dargestellt. Die Klebstoffstreifen 6 bilden ein Klebstoffmuster, welches in einem an das zweite Anschlussende 4 angrenzenden Bereich c₂ einen geringeren Abstand s zwischen benachbarten Klebstoffstreifen 6 aufweist als in einem an das erste Anschlussende 3 angrenzenden Bereich c₁. Die Bereiche c₁, c₂ umfassen jeweils zumindest zwei Klebstoffstreifen 6 mit einem zwischen den Klebstoffstreifen angeordneten klebstofffreien Zwischenraum. In dem in den Fig. 3 und 4 dargestellten Ausführungsbeispiel haben die Klebstoffstreifen 6 eine konstante Breite b von beispielsweise 1,0 mm, wobei die Breite b in einem Wertebereich zwischen 0,5 mm und 1,5 mm festgelegt wird. Der Abstand s zwischen den Klebstoffstreifen 6 ändert sich in Abhängigkeit der Breite B des Laminats und weist in Abhängigkeit der Breite des Laminats festgelegte Werte zwischen 0,5 mm und 2,0 mm auf.

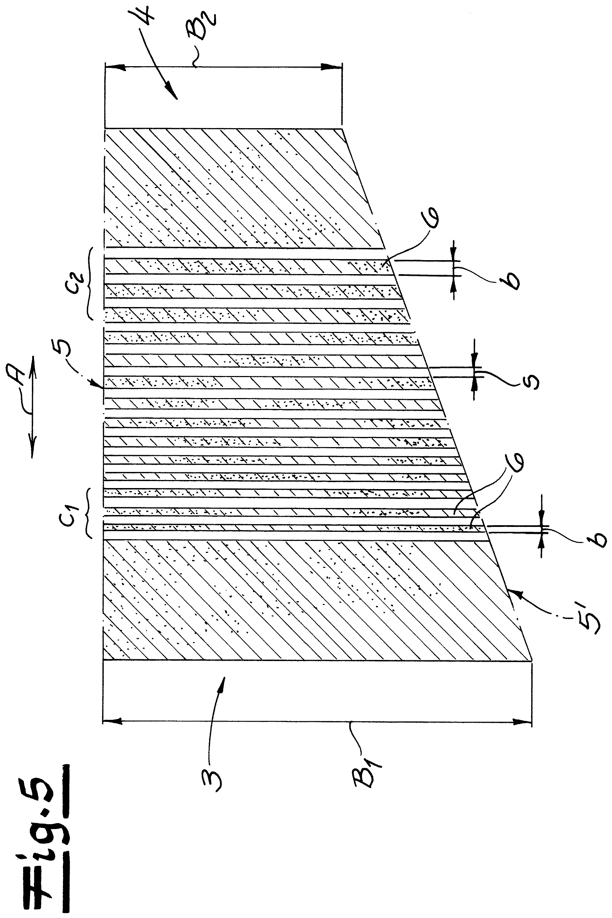

Gemäß einer in Fig. 5 dargestellten Ausführungsvariante ist der Abstand s zwischen den Klebstoffstreifen 6 konstant und die Breite b der Klebstoffstreifen 6 unterschiedlich. In einem an das zweite Anschlussende 4 angrenzenden Bereich c₂ sind die Klebstoffstreifen 6 breiter als in dem an das erste Anschlussende 3 angrenzenden Bereich c₁.

Die in den Fig. 3 bis 5 dargestellten Maßnahmen können auch miteinander kombiniert werden. Erfindungsgemäß ändert sich der Flächenanteil der aus den Klebstoffstreifen 6 gebildeten Klebefläche und der Grundfläche, welche die Klebefläche und die klebstofffreien Flächen umfasst, in Abhängigkeit der Breite B des Laminats. Dabei nimmt der Flächenanteil der Klebefläche bezogen auf die Grundfläche mit kleiner werdender Breite B des Laminats zu. Vorzugsweise ist die Breite b der Klebstoffstreifen 6 und/oder der Abstand s zwischen benachbarten Klebstoffstreifen 6 in Abhängigkeit der Breite B des Laminats so festgelegt, dass die Dehnung des elastischen Bereiches c bei einer Verstreckung des Laminats in Zugrichtung im Wesentlichen einheitlich ist. Die Klebstoffverteilung ist erfindungsgemäß abhängig von der Außengeometrie der Windelkomponente und führt zu einer Vereinheitlichung der Dehnung der Windelkomponente, so dass sich bei einer Dehnung der Windelkomponente in Zugrichtung der elastische Bereich c₂ nahe des zweiten Anschlussendes 4 ebenso stark dehnt wie der an das erste Anschlussende angrenzende elastische Bereich c₁. Im Ergebnis resultiert eine bessere Verteilung der Performance der Windelkomponente.

Der elastische Bereich c des Laminats ist mechanisch aktiviert. Zur mechanischen Aktivierung durchläuft das Laminat eine Profilwalzenanordnung, wobei das Laminat im Walzenspalt zwischen den Profilwalzen lokal überstreckt wird. Die mechanische Aktivierung ist an dem fertigen Produkt sichtbar in Form sogenannter Aktivierungsstreifen. Die Aktivierungsstreifen erstrecken sich parallel zu den Klebstoffstreifen 6 und bewirken eine Verbesserung der Dehnbarkeit des Materials.

Die Anschlussenden 3, 4 des Laminats sind als nicht elastische Zonen a₁, a₂ ausgebildet, wobei der Schichtenaufbau des Laminats an den beiden Anschlussenden 3, 4 unterschiedlich ist. Das erste Anschlussende 3, welches eine große Breite B₁ aufweist und an einer Windel befestigbar ist, ist weich und flexibel und setzt sich lediglich aus den beiden miteinander verbundenen Trägerschichten 1 aus Nonwoven zusammen. Die elastische Folie 2 endet vor dem ersten Anschlussende 3. In der nicht elastischen Zone a₁ sind die Schichten 1, 2 flächig miteinander verbunden. Die flächige Klebeverbindung erstreckt sich über den Rand der elastischen Folie 2 hinaus bis in einen randnahen Bereich der elastischen Folie 2. Das zweite Anschlussende, an dem ein Verschlusselement, z. B. in Form eines Tapes befestigbar ist, ist durch eine Verstärkungsschicht 7 versteift, die in einem Überlappungsbereich an dem mit der einkaschierten elastischen Folie 2 versehenen Abschnitt der Windelkomponente verankert ist. Der Überlappungsbereich kann etwa 2 mm bis 10 mm breit sein. Die Verstärkungsschicht 7 besteht aus einem Polymer, welches sich im Vergleich zu den mechanischen Eigenschaften der elastischen Folie 2 durch eine größere Biegesteifigkeit und hohe Zugfestigkeit auszeichnet.

## Patentansprüche

1. Elastische Windelkomponente bestehend aus einem Laminat, welches zumindest eine Trägerschicht (1) und eine mit der Trägerschicht (1) verklebte elastische Folie (2) aufweist und zumindest im Bereich der elastischen Folie (2) in einer Zugrichtung (A) elastisch dehnbar ist,
wobei das Laminat ein erstes Anschlussende (3) zur Befestigung an einer Windel und ein zweites Anschlussende (4) für ein Verschlusselement aufweist,
wobei das zweite Anschlussende (4) eine geringere Breite (B₂) quer zur Zugrichtung (A) aufweist als das erste Anschlussende (3) und
wobei die Trägerschicht (1) in einem elastischen Bereich (c) zwischen den beiden Anschlussenden (3, 4) mit der elastischen Folie (2) durch eine Mehrzahl paralleler Klebstoffstreifen (6), die sich quer zur Zugrichtung (A) erstrecken, verbunden ist,
wobei die Klebstoffstreifen (6) ein Klebstoffmuster bilden, welches in einem an das zweite Anschlussende (4) angrenzenden Bereich (c₂) breitere Klebstoffstreifen (6) und/oder einen geringeren Abstand (s) zwischen benachbarten Klebstoffstreifen (6) aufweist als in einem an das erste Anschlussende (3) angrenzenden Bereich (c₁), **dadurch gekennzeichnet, dass** die Breite (B) der Klebstoffstreifen (6) und/oder der Abstand (s) zwischen benachbarten Klebstoffstreifen (6) in Abhängigkeit der Breite (B) des Laminats so festgelegt ist, dass die Dehnung des elastischen Bereiches (c) bei einer Verstreckung des Laminats in Zugrichtung im Wesentlichen einheitlich ist.

2. Elastische Windelkomponente nach Anspruch 1, **dadurch gekennzeichnet, dass** der Flächenanteil der aus den Klebstoffstreifen (6) gebildeten Klebefläche bezogen auf die aus der Klebefläche und den klebstofffreien Flächen gebildete Grundfläche sich in Abhängigkeit der Breite (B) des Laminats ändert, wobei der Flächenanteil der Klebefläche mit kleiner werdender Laminatbreite (B) zunimmt.

3. Elastische Windelkomponente nach einem der Ansprüche 1 bis 2, **dadurch gekennzeichnet, dass** die Klebstoffstreifen (6) eine vorzugsweise konstante Breite (b) zwischen 0,5 mm und 1,5 mm aufweisen und dass die Abstände (s) zwischen den Klebstoffstreifen (6) sich in Abhängigkeit der Laminatbreite ändern und Werte zwischen 0,5 mm und 2,0 mm aufweisen.

4. Elastische Windelkomponente nach einem der Ansprüche 1 bis 3, **dadurch gekennzeichnet, dass** der elastische Bereich (c) des Laminats mechanisch aktiviert ist.

5. Elastische Windelkomponente nach einem der Ansprüche 1 bis 4, **dadurch gekennzeichnet, dass** die Trägerschicht (1) aus einem Nonwoven besteht.

6. Elastische Windelkomponente nach einem der Ansprüche 1 bis 5, **dadurch gekennzeichnet, dass** die elastische Folie (2) zwischen zwei aus Nonwoven bestehenden Trägerschichten (1) angeordnet ist.

7. Elastische Windelkomponente nach einem der Ansprüche 1 bis 6, **dadurch gekennzeichnet, dass** die Anschlussenden (3, 4) als nichtelastische Zonen ausgebildet sind.

## Claims

1. Elastic nappy component consisting of a laminate which comprises at least one carrier layer (1) and an elastic film (2) adhesively bonded to the carrier layer (1) and which is elastically stretchable at least in the area of the elastic film (2) in a tensile direction (A),
wherein the laminate has a first connecting end (3) for fastening to a nappy and a second connecting end (4) for a closure element,
wherein the second connecting end (4) has a smaller width (B₂) transverse to the tensile direction (A) than the first connecting end (3) and
wherein in an elastic region (c) between the two connecting ends (3, 4), the carrier layer (1) is connected to the elastic film (2) by a multiplicity of parallel adhesive strips (6) which extend parallel to the tensile direction (A),
wherein the adhesive strips (6) form an adhesive pattern which in a region (c₂) adjoining the second connecting end (4) have broader adhesive strips (6) and/or a smaller spacing (s) between adjacent adhesive strips (6) than in a region (c₁) adjoining the first connecting end (3), **characterized in that** the width (B) of the adhesive strips (6) and/or the spacing (s) between adjacent adhesive strips (6) is specified depending on the width (B) of the laminate so that the stretching of the elastic region (c) during a stretching of the laminate in the tensile direction is substantially uniform.

2. The elastic nappy component according to claim 1, **characterized in that** the surface fraction of the adhesive surface formed by the adhesive strips (6) relative to the base surface formed from the adhesive surface and the adhesive-free surfaces varies depending on the width (B) of the laminate, wherein the surface fraction of the adhesive surface increases as the laminate width (B) becomes smaller.

3. The elastic nappy component according to any one of claims 1 to 2, **characterized in that** the adhesive strips (6) have a preferably constant width (b) between 0.5 mm and 1.5 mm and that the spacings (s) between the adhesive strips (6) vary depending on the laminate width and have values between 0.5 mm and 2.0 mm.

4. The elastic nappy component according to any one of claims 1 to 3, **characterized in that** the elastic region (c) of the laminate is mechanically activated.

5. The elastic nappy component according to any one of claims 1 to 4, **characterized in that** the carrier layer (1) consists of a nonwoven.

6. The elastic nappy component according to any one of claims 1 to 5, **characterized in that** the elastic film (2) is arranged between two carrier layers (1) consisting of nonwoven.

7. The elastic nappy component according to any one of claims 1 to 6, **characterized in that** the connecting ends (3, 4) are configured as non-elastic zones.

## Revendications

1. Composante élastique de couche-culotte composée d'un stratifié qui présente au moins une couche porteuse (1) et un film élastique (2) collé avec la couche porteuse (1) et est extensible élastiquement dans un sens de traction (A), au moins au niveau du film élastique (2),
dans lequel le stratifié présente une première extrémité de raccord (3) destinée à la fixation sur une couche-culotte et une seconde extrémité de raccord (4) pour un élément de fermeture,
dans lequel la seconde extrémité de raccord (4) présente une largeur plus étroite (B₂) transversalement au sens de traction (A) que la première extrémité de raccord (3) et
dans lequel la couche porteuse (1), dans une partie élastique (c) entre les deux extrémités de raccord (3, 4), est reliée au film élastique (2) par une pluralité de bandes d'adhésif (6) parallèles qui s'étendent transversalement au sens de traction (A),
dans lequel les bandes d'adhésif (6) forment un motif d'adhésif qui présente des bandes d'adhésif (6) plus larges dans une partie (C₂) adjacente à la seconde extrémité de raccord (4) et/ou un écart plus faible (s) entre des bandes d'adhésif (6) voisines que dans une partie (c₁) adjacente à la première extrémité de raccord (3), **caractérisée en ce que** la largeur (B) des bandes d'adhésif (6) et/ou l'écart (s) entre des bandes d'adhésif (6) voisines est déterminée de telle façon en fonction de la largeur (B) du stratifié que l'allongement de la partie élastique (c) est essentiellement uniforme lors d'un étirage du stratifié dans le sens de traction.

2. Composante élastique de couche-culotte selon la revendication 1, **caractérisée en ce que** la fraction de surface de la surface d'adhésif formée à partir des bandes d'adhésif (6) rapportée à la surface de base formée par la surface d'adhésif et les surfaces exemptes d'adhésif se modifie en fonction de la largeur (B) du stratifié, dans lequel la fraction de surface de la surface d'adhésif augmente à mesure que la largeur (B) de stratifié réduit.

3. Composante élastique de couche-culotte selon l'une des revendications 1 à 2, **caractérisée en ce que** les bandes d'adhésif (6) présentent une largeur (b) de préférence constante entre 0,5 mm et 1,5 mm et que les écarts (s) entre les bandes d'adhésif (6) se modifient en fonction de la largeur du stratifié et présentent des valeurs entre 0,5 mm et 2 mm.

4. Composante élastique de couche-culotte selon l'une des revendications 1 à 3, **caractérisée en ce que** la partie élastique (c) du stratifié est activée mécaniquement.

5. Composante élastique de couche-culotte selon l'une des revendications 1 à 4, **caractérisée en ce que** la couche porteuse (1) est composée d'un non-tissé.

6. Composante élastique de couche-culotte selon l'une des revendications 1 à 5, **caractérisée en ce que** le film élastique (2) est disposé entre deux couches porteuses (1) composées de non-tissé.

7. Composante élastique de couche-culotte selon l'une des revendications 1 à 6, **caractérisée en ce que** les extrémités de raccord (3, 4) sont formées en tant que zones non élastiques.
